# EUROPEAN PATENT SPECIFICATION

(11) **EP 4 132 386 B1**
(45) Date of publication and mention of the grant of the patent: **26.11.2025**
(21) Application number: 21783858.0
(22) Date of filing: 05.04.2021
(51) Int. Cl.: A61B 17/221, A61B 18/02, A61B 17/22

(54) **BLOOD CLOT RETRIEVAL SYSTEMS**
SYSTEME ZUR BLUTGERINNSELGEWINNUNG
SYSTÈMES DE RÉCUPÉRATION DE CAILLOTS DE SANG

(30) Priority: 08.04.2020 US 202063007215 P
(43) Date of publication of application: 15.02.2023
(60) Divisional application: 25205325.1
(73) Proprietor: Mayo Foundation for Medical Education and Research, Rochester, Minnesota 55905 (US)
(72) Inventor: ASIRVATHAM, Samuel J., Rochester, Minnesota 55906-8971 (US); ALKHOULI, Mohamad A., Rochester, Minnesota 55902-3495 (US)
(74) Representative: Fish & Richardson P.C.
(86) International application number: PCT/US2021/025759
(87) International publication number: WO 2021/207066

(56) References cited:
- WO-A1-2010/061376
- WO-A1-2013/008233
- WO-A1-2019/212984
- US-A1- 2012 150 107
- US-A1- 2013 018 387
- US-A1- 2015 112 195
- US-A1- 2018 116 704
- US-A1- 2019 209 189
- US-A1- 2019 336 149
- US-B1- 6 468 296

## Description

### CROSS-REFERENCE TO RELATED APPLICATIONS

This application claims the benefit of U.S. Provisional Application Serial No. 63/007,215 filed April 8, 2020.

### BACKGROUND

### 1. Technical Field

This document relates to systems and methods for retrieving blood clots. For example, this document relates to catheter-based cryogenic devices that are used to retrieve and remove blood clots.

### 2. Background Information

Large thrombus burden is associated with worse outcomes in patients admitted with acute myocardial infarction (AMI), venous thromboembolism (VTE), acute ischemic stroke, and acute limb ischemia (ALI). To date, strategies to tackle large thrombus burden have demonstrated modest success. Hence, a large unmet need exists. Over 4 million admissions occurring annually for AMI, VTE, AIS, and ALI in the United States, and up to 20% of them are associated with significant thrombus burden.

Cryogenic (or "cryo") energy is commonly used for ablation of cardiac tissue (e.g., cryogenic ablation of atrial fibrillation). However, in some cases cryogenic catheters may also be used to 'adhere' to the surrounding tissue structures while activated.

WO 2019/212984 A1 describes a system including an elongate, flexible tubular body, having a proximal end, a distal end, and a tubular side wall defining at least one lumen extending axially there through; an axial restraint carried by the side wall and exposed to the lumen; a rotatable core wire extendable through the lumen, the core wire having a distal end; a limit carried by the core wire, the limit having a bearing surface for rotatably engaging the restraint; and an agitator tip on the distal end of the core wire. The limit and the restraint are engageable to permit rotation of the core wire but limit distal advance of the tip relative to the tubular body.

WO 2013/008233 A1 describes a retrieval apparatus including a snare and a snare control assembly. The snare has a proximal section and a distal section, and comprises a plurality of filaments extending from a proximal end of the proximal section towards the distal section, and then returning to the proximal end to form a plurality of loops. In the deployed state, the loops are interlaced to each other within the proximal section and are free and not interleaved within the distal section. Segments of the filaments of the distal section are bent with respect to segments of the filaments of the proximal section such that the retrieval snare in the contracted state forms a hollow cavity extending from the distal section towards the proximal section.

### SUMMARY

The invention is defined by the features of the independent claims. Embodiments thereof are defined by the sub-features of the dependent claims.

This document describes systems and methods for retrieving blood clots. For example, this document describes catheter-based cryogenic devices that are used to retrieve and remove blood clots.

In one embodiment, this disclosure is directed to a blood clot retrieval system. Such a blood clot retrieval system includes: a cryogenic catheter configured for intravascular use and defining a coolant flow loop, and a wire that is slidably disposable within a lumen of the cryogenic catheter and distally extendable from a distal tip of the cryogenic catheter. The coolant flow loop is arranged to cool at least a distal end portion of the wire when a coolant is within the coolant flow loop and the wire is within the lumen.

Such a blood clot retrieval system may optionally include one or more of the following features. The distal end portion of the wire may comprise a spiral. The distal end portion of the wire may comprise a corkscrew configuration. The distal end portion of the wire may comprise multiple separate loops. The distal end portion of the wire may comprise a basket configuration.

In another embodiment, this disclosure is directed to a blood clot retrieval system. The blood clot retrieval system includes: (i) a catheter configured for intravascular use; (ii) a wire that is slidably disposable within a lumen of the catheter; and (iii) at least two Peltier devices attached to a distal end portion of the wire and spaced apart from each other. Activation of the Peltier devices cools a first portion of the wire located between the Peltier devices and heats a second portion of the wire located between the Peltier devices. The first portion of the wire is uninsulated and the second portion of the wire is insulated.

Such a blood clot retrieval system may optionally include one or more of the following features. The distal end portion of the wire may comprise a spiral. The distal end portion of the wire may comprise a corkscrew configuration. The distal end portion of the wire may comprise multiple separate loops.

In another aspect, this disclosure is directed to a blood clot retrieval system that includes a cryogenic catheter configured for intravascular use. The cryogenic catheter comprises a coolant flow loop. Causing coolant to flow through the coolant flow loop results in cooling a distal end portion of the coolant flow loop.

In another aspect, this disclosure is directed to a method for retrieving thrombus from a blood vessel. The method includes: (i) advancing, within the blood vessel, any one of the blood clot retrieval systems described herein; (ii) cooling the distal end portion of the wire or the coolant flow loop; (iii) causing adherence of the cooled distal end portion of the wire to the thrombus; and (iv) removing the blood clot retrieval system and the thrombus from the blood vessel.

Particular embodiments of the subject matter described in this document can be implemented to realize one or more of the following advantages. The devices and methods described herein can be used to remove blood clots from the vasculature of a patient so that blood flow is advantageously restored. In some embodiments, thrombus can be removed in a minimally invasive fashion using the devices and methods provided herein. Such minimally invasive techniques can reduce recovery times, patient discomfort, and treatment costs.

The systems and methods described herein can be used for any and all types of intravascular clot or embolus removal treatment such as, but not limited to, pulmonary embolism or thrombosis, deep venous embolism or thrombosis, arterial embolism or thrombosis, coronary embolism or thrombosis, bypass graft embolism thrombosis, renal vein embolism or thrombosis, portal vein embolism or thrombosis, intracardiac embolism thrombosis, carotid and cerebral vessel embolism or thrombosis. Unless otherwise defined, all technical and scientific terms used herein have the same meaning as commonly understood by one of ordinary skill in the art to which this invention pertains.

The details of one or more embodiments of the invention are set forth in the accompanying drawings and the description herein. Other features, objects, and advantages of the invention will be apparent from the description and drawings, and from the claims.

In this specification the non-SI unit 'inch' is used, which may be converted to the SI or metric unit according to the following conversion: 1 inch ≅ 25.4 mm.

### DESCRIPTION OF THE DRAWINGS

FIG. 1 illustrates blood vessel containing a blood clot thrombus.
FIG. 2 illustrates a first step for using an example thrombus retrieval system for removing the thrombus of FIG. 1.
FIG. 3 illustrates a second step for using the thrombus retrieval system of FIG. 2.
FIG. 4 illustrates a third step for using the thrombus retrieval system of FIG. 2.
FIG. 5 illustrates a fourth step for using the thrombus retrieval system of FIG. 2.
FIG. 6 illustrates the blood vessel after removal of the thrombus and removal of the thrombus retrieval system of FIG. 2.
FIG. 7 illustrates another embodiment of a thrombus retrieval system.
FIG. 8 illustrates another embodiment of a thrombus retrieval system.
FIG. 9 illustrates another embodiment of a thrombus retrieval system.
FIG. 10 illustrates another embodiment of a thrombus retrieval system.
FIG. 11 illustrates a first step for using another example thrombus retrieval system.
FIG. 12 illustrates a second step for using the thrombus retrieval system of FIG. 11.
FIG. 13 illustrates a third step for using the thrombus retrieval system of FIG. 11.
FIG. 14 illustrates a fourth step for using the thrombus retrieval system of FIG. 11.
FIG. 15 illustrates another alternative embodiment of a thrombus retrieval system.
FIG. 16 illustrates another alternative embodiment of a thrombus retrieval system.
FIG. 17 illustrates another alternative embodiment of a thrombus retrieval system.
FIG. 18 illustrates a first step for using another alternative example of a thrombus retrieval system.
FIG. 19 illustrates a second step for using the thrombus retrieval system of FIG. 18.
FIG. 20 illustrates another alternative example of a thrombus retrieval system.
FIG. 21 illustrates another alternative example of a thrombus retrieval system.
FIG. 22 illustrates another alternative example of a thrombus retrieval system.
FIG. 23 illustrates another alternative example of a thrombus retrieval system.
FIG. 24 illustrates another alternative example of a thrombus retrieval system.
FIG. 25 illustrates another alternative example of a thrombus retrieval system.
FIGs. 26-28 illustrate another alternative example of a thrombus retrieval system.
FIGs. 29-31 illustrate another alternative example of a thrombus retrieval system.
FIGs. 32-34 illustrate another alternative example of a thrombus retrieval system.
FIG. 35 illustrates another alternative example of a thrombus retrieval system.
FIGs. 36 and 37 illustrate another alternative example of a thrombus retrieval system.
FIG. 38 illustrates another alternative example of a thrombus retrieval system.
FIG. 39 illustrates another alternative example of a thrombus retrieval system.

Like reference numbers represent corresponding parts throughout.

### DETAILED DESCRIPTION

This document describes systems and methods for retrieving blood clots. For example, this document describes catheter-based cryogenic devices that are used to retrieve and remove blood clots. The systems and methods provided herein can be used to remove blood clots (or "thrombus") from all types of blood vessels including, but not limited to, peripheral vessels, coronary arteries, cerebral arteries, and any other type of blood vessel that can contain blood clot thrombi.

Novel application of cryogenic energy in a form of a 'cryogenic wire' may allow safe and effective thrombus removal, and hence may facilitate flow restoration in coronary and non-coronary vessels with significant thrombus burden.

This disclosure describes cryogenic wire devices that allow thrombus removal from the vasculature. The cryogenic wire devices are suitable for intra-vascular thrombus removal. Multiple embodiments of cryogenic thrombus retrieval devices and systems are described herein. In some embodiments, without limitation, a 0.014 inch (0.356 mm), 0.018 inch (0.457 mm), 0.025 inch (0.635 mm), 0.032 inch (0.813 mm), or 0.035 inch (0.889 mm) wire can be cooled within and then extended from the tip of the 6-8 French (2-2.667 mm) coolant-based cryogenic catheter. With this system/method, a 6-8 French (2-2.667 mm) coolant-based cryogenic catheter will be advanced to the vessel ostium via a guiding sheath. The extendable wire with various configurations will be then advanced into the clotted segment. The cryogenic system will be activated by delivering a coolant to the distal tip. The cryogenic energy will transfer from the tip of the cryogenic catheter into the extendable wire. The extendable wire will thus by activated 'cryogenic' which will help the thrombus to adhere to the wire. The wire will be removed back into the delivery catheter/sheath along with the cryogenic catheter, achieving thrombus removal.

Another type of device/system embodiment described herein includes, for example, 0.014 inch (0.356 mm), 0.018 inch (0.457 mm), 0.025 inch (0.635 mm), 0.032 inch (0.813 mm), or 0.035 inch (0.889 mm) cryogenic wire using electro-thermal cryogenic energy (e.g., the Peltier Effect). With this method, two Peltier electrodes will be installed on one or more wires. The area between the two Peltier devices will be the 'active' thermoelectrical 'Cryo' portion of the wire. The proximal end of the wire will be connected to an electrical current source that delivers electricity to the Peltier devices. Conversion of the electrical current to thermal energy between the two Peltier electrodes allows cooling of this specific portion of the wire, leading to its activation as a 'Cryo' wire. This thermoelectrical energy activation allows the clot adjacent to the 'cooled' wire to adhere to it. The wire can then be pulled back into the guiding catheter and removed from the vasculature of the body. Because electro-thermal cryogenic energy leads to emission of both heating and cooling energy, the part of the active portion of the wire that will emit heat energy will be insulated in some embodiments.

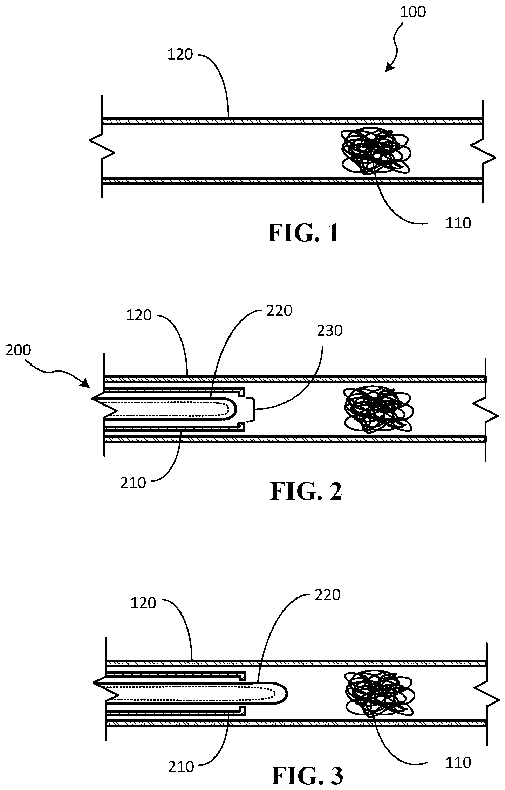

FIG. 1 shows an example of clogged vasculature 100 where a thrombus 110 is lodged within a blood vessel 120 (e.g., vein, artery). A thrombus 110 is a body of coagulated blood. The blood vessel 120 may be any example of the vascular system.

FIG. 2 shows a first example of a cryogenic catheter 200 being extended longitudinally into the blood vessel 120 in proximity to the thrombus 110. In the first example, the cryogenic catheter 200 includes a guide sheath 210 which surrounds the cryogenic catheter 220. The diameter of the guide sheath 210 *D_{G}* may be smaller than the diameter of the blood vessel 120 (e.g., *D_{G}* ≤ *D_{V}*) but larger than the diameter of the cryogenic catheter 220 *D_{C}* (e.g., *D_{C}* ≤ *D_{G}*)*.* In some examples, the cryogenic catheter 220 may have a diameter *D_{C}* between 6 and 8 French (e.g., 2 mm ≤ *D_{C} ≤* 2.3 mm). The guide sheath 210 includes a guide sheath aperture 230 in the distal end. The diameter of the guide sheath aperture 230 *D_{A}* may be less than the diameter of the guide sheath 210 and larger than the diameter of the cryogenic catheter 220 (e.g., *D_{C}* ≤*D_{A}* ≤*D_{G}*). For the purposes of the descriptions herein, the components of the cryogenic catheter 200 to the right in FIG. 2 are deemed as oriented distal to a clinician, and components to the left are deemed as oriented proximally to the clinician.

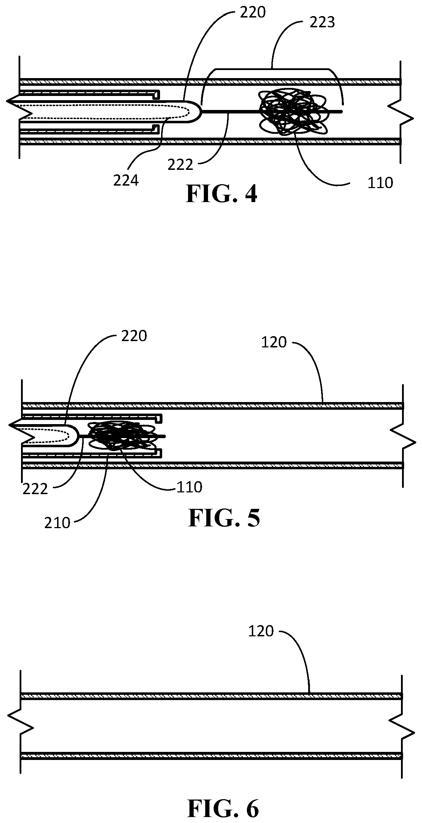

FIG. 3 shows the cryogenic catheter 220 extending through the guide sheath 210 and into the luminal space of the blood vessel 120 in proximity to the thrombus 110. This step is optional. In some examples, the cryogenic catheter 220 can remain in the sheath 210 while the cryogenic wire 222 (FIG. 4) is caused to extend distally from the cryogenic catheter 220.

Referring also to FIG. 4, a cryogenic wire 222 is shown slidably disposed past the distal tip of the cryogenic catheter 220. The cryogenic catheter 220 may be advanced distally into the luminal space of the blood vessel 120 far enough such that the thrombus 110 is within a distance less than the length 223 of the cryogenic wire.

The cryogenic wire 222 may be made of any material with high thermal conductivity (e.g., silver, copper, aluminum, steel, etc.) to enable rapid cooling. The cryogenic wire 222 may have any diameter *D_{W}* but an example range may be between 0.014" and 0.018" (e.g., 0.014" ≤ *D_{W}* ≤ 0.018"). The cryogenic wire 222 may be of any desirable length but in some examples may extend to the proximal end of the cryogenic catheter 220. In some examples, a cryogenic wire 222 that extends to the proximal end of the cryogenic catheter 210 may by steered by the clinician through the use of a steering tool. The cryogenic wire 222 may be slidably disposable past the cryogenic catheter 210 by any length 223 but example lengths 223 the cryogenic wire 222 may extend past the cryogenic catheter 210 may be between 2 cm and 4 cm (e.g., 2 cm, 3 cm, 4 cm).

When the cryogenic catheter 220 has been advanced to within a distance less than the length 223 of the cryogenic wire 222, the cryogenic wire 222 may be extended into the thrombus 110. In some examples, the cryogenic wire 222 may be extended through the application of an electrical current. The cryogenic wire 222 is connected to a coolant flow loop 224 capable of delivering a coolant to the cryogenic wire 222. In some examples, the cryogenic wire 222 may be slidably disposed through the application of the coolant.

Once the cryogenic wire 222 has been disposed into the thrombus 110, the clinician may activate the cryogenic catheter 220 to deliver coolant from within the coolant flow loop 224 to the cryogenic wire 222, rapidly cooling the cryogenic wire 222. In some examples, the coolant may be liquid nitrogen but any desirable coolant may be used (e.g., liquid nitrous oxide). In some examples, the cryogenic wire may be cooled to -70° C but a range of temperatures may be considered (e.g., -10° C, -30° C, - 50° C, -70° C, -90° C). The cryogenic wire 222 may be left in place within the thrombus 110 for any desirable length of time. In some examples, the cryogenic wire 222 may be left within the thrombus 110 for between 5 to 60 seconds (e.g., 5 s, 10 s, 20 s, 40 s, 60 s). While the cooled cryogenic wire 222 is in contact with the thrombus 110, the thrombus 110 dehydrates and freezes to the distal end of the cryogenic wire 222. Once removed from the body, the cryogenic wire 222 can be deactivated (unfrozen) and the clot adherent to the cryogenic wire 222 can then be manually removed. The cryogenic wire 222 may then be reused to remove further clots from the same vessel or different vessels.

As shown in FIG. 5, once the thrombus 110 has become adhered to the distal end of the cryogenic wire 222, the cryogenic wire 222 may be retracted into the cryogenic catheter 220 to a distance at which the thrombus is in near proximity to the distal tip of the cryogenic catheter 220. The cryogenic catheter 220 may then be withdrawn into the guide sheath 210 such that both the cryogenic catheter 220 and the thrombus 110 frozen to the cryogenic wire 222 are withdrawn partially or fully through the guide sheath aperture 230 and into the luminal area of the guide sheath 210. The cryogenic catheter 200 may then be removed from the blood vessel 120.

Referring also to FIG. 6, this depicts the blood vessel 120 after the guide sheath 210 and thrombus 110 removed.

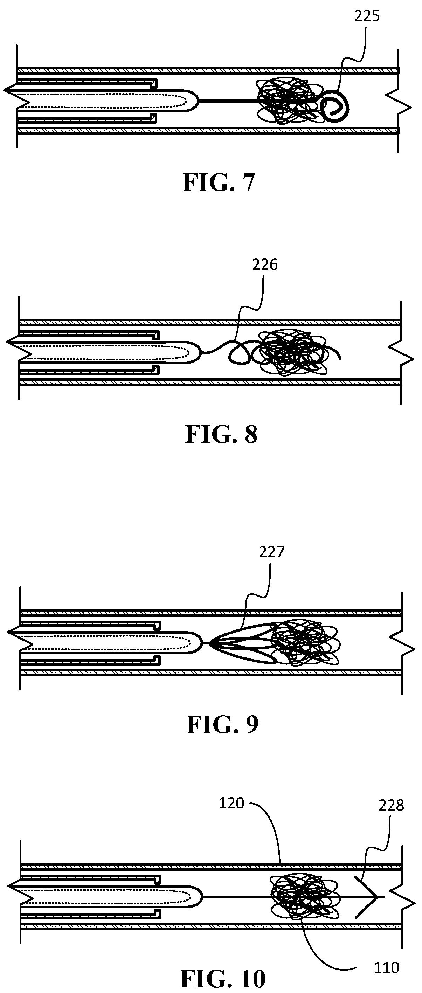

FIGS. 7 to 9 depict alternative embodiments for the cryogenic wire 222. Alternative embodiments may be designed to provide increased contact surface between the cryogenic wire 222 and the thrombus 110.

FIG. 7 depicts an alternative embodiment of the cryogenic wire 222 with a distal end portion configured as a 'spiral' coil 225. The 'spiral' coil 225 may be placed at the distal end of the cryogenic wire 222 and may be coiled such that the plane of the 'spiral' coil 225 is aligned longitudinally with the longitudinal axis of the cryogenic wire 222. In alternative embodiments, the plane of the 'spiral' coil 225 may be set at an angular offset of up to 90° from the longitudinal axis of the cryogenic wire 222 (e.g., 1°, 10°, 30°, 50°, 70°, 90°).

FIG. 8 depicts an alternative embodiment of the cryogenic wire 222 with a distal end portion configured in a 'corkscrew' formation 226. FIG. 8 depicts a cryogenic wire 222 in a 'corkscrew' formation 226 with four turns, but other embodiments may have one or more turns (e.g., one turn, two turns, three turns, four turns). The turns may be predominantly located near the distal end of the cryogenic wire 222 or evenly spaced along the length of the cryogenic wire 222, as shown. The radius of gyration of the 'corkscrew' formation may be more than the radius of the cryogenic wire 222 and less than the radius of the guide sheath aperture 230.

FIG. 9 depicts an alternative embodiment of the cryogenic wire 222 in a looped 'snare' formation 227. The 'snare' formation 227 may have three loops, as shown, but other embodiments may have one or more loops (e.g., one loop, two loops, three loops, four loops, etc.). The 'snare' formation 227 loops may be circular or elliptical. The 'snare' formation 227 loops may have a common connection point to the cryogenic wire 222 or they may have individual connection points spaced along the length 223 of the cryogenic wire 222. The 'snare' formation 227 loops may be designed to encircle the thrombus 110 or to be implanted in or around the thrombus 110.

FIG. 10 depicts an alternative embodiment of the cryogenic wire 222 in an 'umbrella' formation 228. The 'umbrella' formation 228 may be of a fixed width or it may be deployable to open upon actuation by the clinician. In some embodiments of the 'umbrella' formation 228, deployment may be actuated by the clinician on the distal side of the thrombus 110 and may be collapsible upon a second clinician actuation. In other embodiments, the 'umbrella' formation 228 may be actuated through application of the coolant. In other embodiments, the 'umbrella' formation 228 may be actuated through application of an electrical current. The deployable embodiment of the 'umbrella' formation 228 may serve to keep the cryogenic wire 222 centered within the blood vessel 120. The clinician-actuated collapse of the deployable embodiment of the 'umbrella' formation 228 may serve to aid in cryogenic wire 222 incorporation and retrieval of the thrombus 110.

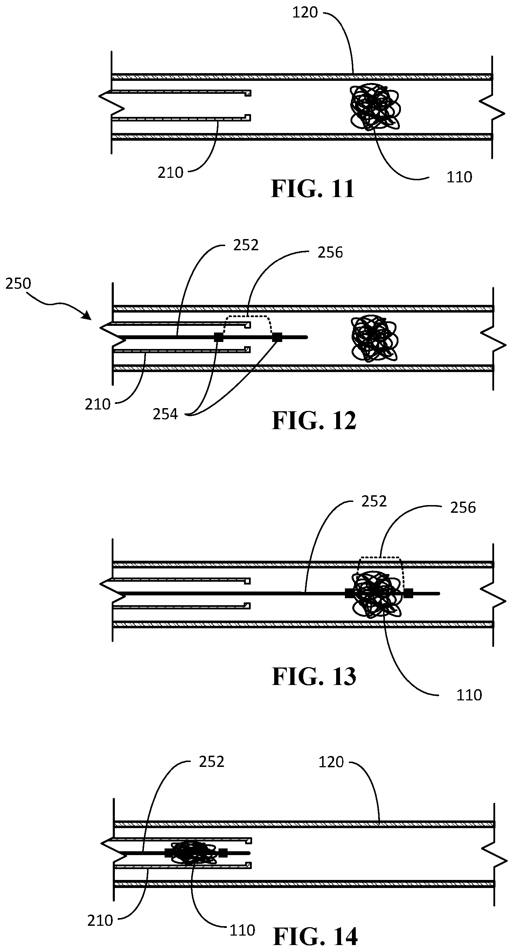

In an alternative embodiment, a guide sheath 210 may be used for thrombus 110 retrieval. FIG. 11 depicts the guide sheath 210 being disposed longitudinally into the blood vessel 120 in proximity to the thrombus 110.

Referring also to FIG. 12, a Peltier cryogenic catheter 250 is slidably extendable relative to the guide sheath 210. The Peltier cryogenic catheter 250 may consist of an insulated Peltier cryogenic wire 252, two Peltier electrodes 254 that are spaced apart from each other, and a partially uninsulated portion 256 between the two Peltier electrodes 254. The Peltier cryogenic wire 252 may be made of any material capable of electrical conduction and having high thermal conductance (e.g., silver, copper, aluminum, steel, etc.). Similarly, the Peltier electrodes 254 (which may comprise semiconductors) may be made of any material capable of electrical conduction and having high thermal conductance (e.g., silver, copper, aluminum, steel, etc.). The Peltier electrodes 254 may be made of the same material as the Peltier cryogenic wire 252 or they may be made of a different material. The Peltier electrodes 254 may be made of the same material or they may be made of a different material from each other.

The proximal end of the Peltier cryogenic wire 252 may extend the length of the guide sheath 210 and be connected to an electrical source (not shown) capable of being activated by the clinician. Upon activation of the electrical source, an electrical current may be applied to the Peltier cryogenic wire 252. The length of the Peltier cryogenic wire 252 may be insulated for the length of the wire except for the inter-electrode portion 256 which need only be insulated against heat. The cold side of the Peltier cryogenic wire 252 may be left exposed so as to expose the thrombus 110 to cold temperatures when in contact with the thrombus 110. The inter-electrode portion 256 may be of any length but in some example embodiments, may be between 1 cm and 4 cm (e.g., 1 cm, 2 cm, 3 cm, 4 cm, etc.)

When the guide sheath 210 has been advanced to within a distance less than the extensible length of the Peltier cryogenic wire 252, the Peltier cryogenic wire 252 may be advanced through the guide sheath aperture.

FIG. 13 depicts the Peltier cryogenic wire 252 inserted into the thrombus 110. The Peltier cryogenic wire 252 may be disposed partially into the thrombus 110 or fully. The Peltier cryogenic wire 252 may be inserted into the thrombus 110 such that the inter-electrode portion 256 is partially or fully within the thrombus 110. The Peltier cryogenic wire 252 may be left in place within the thrombus 110 for any desirable length of time. In some embodiments, the Peltier cryogenic wire 252 may be left within the thrombus 110 for between 5 to 60 seconds (e.g., 5 s, 10 s, 20 s, 40 s, 60 s). While the cooled Peltier cryogenic wire 252 is in contact with the thrombus 110, the thrombus 110 dehydrates and freezes to the exposed inter-electrode portion 256 of the Peltier cryogenic wire 252.

Referring also to FIG. 14, once the thrombus 110 has been frozen to the inter-electrode portion 256 of the Peltier cryogenic wire 252, the Peltier cryogenic wire 252 may be retracted into the guide sheath 210. The Peltier cryogenic wire 252 may be retracted such that both the Peltier cryogenic wire 252 and the thrombus 110 frozen to the Peltier cryogenic wire 252 are withdrawn partially or fully into the luminal area of the guide sheath 210. The guide sheath 210, Peltier cryogenic wire 252, and thrombus 210 may then be removed from the blood vessel 120.

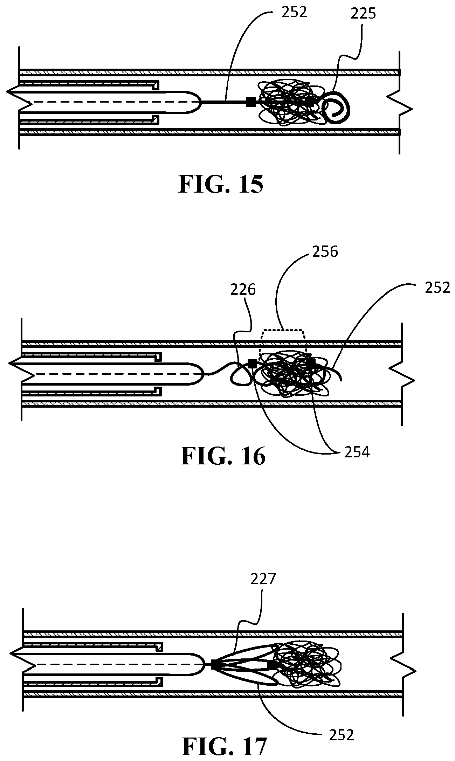

FIGS. 15 to 17 depict alternative embodiments for the Peltier cryogenic wire 252. Alternative embodiments may be designed to provide increased contact surface between the Peltier cryogenic wire 252 and the thrombus 110.

FIG. 15 depicts an alternative embodiment of the Peltier cryogenic wire 252 with a distal end in a 'spiral' coil 225. The 'spiral' coil 225 may be placed at the distal end of the Peltier cryogenic wire 252 and may be coiled such that the plane of the 'spiral' coil 225 is aligned longitudinally with the longitudinal axis of the Peltier cryogenic wire 252. In alternative embodiments, the plane of the 'spiral' coil 225 may be set at an angular offset of up to 90° from the longitudinal axis of the Peltier cryogenic wire 252 (e.g., 1°, 10°, 30°, 50°, 70°, 90°).

FIG. 16 depicts an alternative embodiment of the Peltier cryogenic wire 252 in a 'corkscrew' formation. FIG. 15 depicts a Peltier cryogenic wire 252 in a 'corkscrew' formation with four coils 810, but other embodiments may have one or more coils (e.g., one coil, two coils, three coils, four coils). The coils may be predominantly located near the distal end of the Peltier cryogenic wire 252 or evenly spaced along the length of the Peltier cryogenic wire 252, as shown. The Peltier devices 254 and exposed inter-electrode portion 256 may be placed along the coils, proximally, or distally. The radius of gyration of the coils may be more than the radius of the Peltier cryogenic wire 252 and less than the radius of the guide sheath aperture 230.

FIG. 17 depicts an alternative embodiment of the Peltier cryogenic wire 252 in a 'snare' formation 227 consisting of multiple separate loops. FIG. 16 depicts a Peltier cryogenic wire 252 with three loops, but other embodiments may have one or more loops (e.g., one loop, two loops, three loops, four loops). The loops may be circular or elliptical. The loops may have a common connection point to the Peltier cryogenic wire 252 or they may have connection points spaced along the length of the Peltier cryogenic wire 252. The Peltier devices 254 may be the common connection point to the Peltier cryogenic wire 252 or the Peltier devices 254 may be spaced along the loops. The loops may be designed to encircle the thrombus 110 or to be implanted in the thrombus 110.

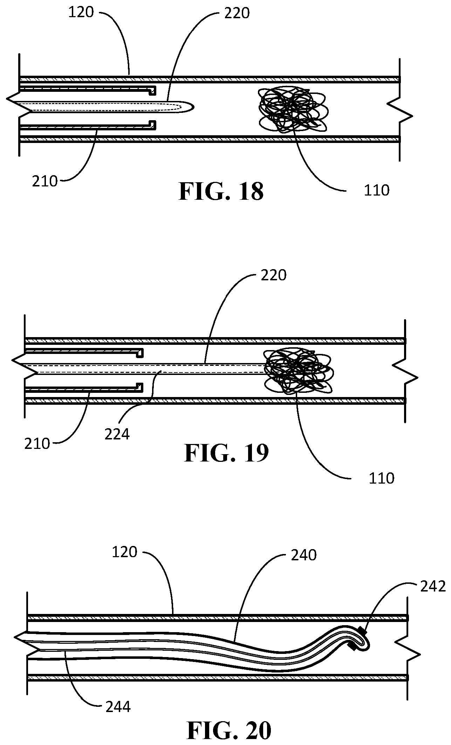

FIGS. 18 and 19 depict an example using only a cryogenic catheter 220 that contains a coolant flow loop 224. In some examples, an optional sheath 210 is also used.

FIG. 18 depicts the cryogenic catheter 220 extending distally into the luminal area of a blood vessel 120 in proximity to a thrombus 110. In some examples, at least a distal end portion of the cryogenic catheter 220 is deflectable/steerable. In some examples, the cryogenic catheter 220 includes two regions that are deflectable/steerable.

Referring also to FIG. 19, the cryogenic catheter 220 may be slidably disposed distally into the thrombus 110. In some examples, the distal end portion of the cryogenic catheter 220 may have one or more electrodes/sensors capable of detecting the presence of tissue (not shown). Once the distal tip of the cryogenic catheter 220 has been slidably disposed into the thrombus 110, the clinician may activate the cryogenic catheter 220 to deliver coolant flow within the coolant flow loop 224 to the distal tip of the cryogenic catheter 220, rapidly cooling the distal tip of the cryogenic catheter 220. In some examples, the coolant may be liquid nitrogen but any desirable coolant may be used (e.g., liquid nitrous oxide). In some examples, the cryogenic wire may be cooled to -70° C but a range of temperatures may be utilized (e.g., -10° C, -30° C, -50° C, -70° C, -90° C). In some examples, the distal tip portion of the cryogenic catheter 220 is uninsulated, while regions of the cryogenic catheter 220 proximal thereto are insulated.

The cryogenic catheter 220 may be left in place within the thrombus 110 for any desirable length of time. In some examples, the cryogenic catheter 220 may be left within the thrombus 110 for between 10 to 60 seconds (e.g., 10 s, 20 s, 40 s, 60 s). While the cooled cryogenic catheter 220 is in contact with the thrombus 110, the thrombus 110 dehydrates and freezes to the distal end of the cryogenic catheter 220. Once the thrombus 110 has been frozen to the distal end of the cryogenic catheter 220, the cryogenic catheter 220 may then be removed from the blood vessel 120.

In alternative examples, the distal end of the cryogenic catheter 220 shown in FIGS. 18 and 19 may be capable of being steered by the clinician. Using a steering knob (not shown) located on the proximal end of the cryogenic catheter 220, a clinician may deform the distal end of the cryogenic catheter 220 to help guide the catheter in proximity to the thrombus 110. In some examples, the cryogenic catheter 220 may be steered through azimuthal tilting of the distal end of the cryogenic catheter 220.

FIG. 20 depicts another example cryogenic catheter 240. The distal end portion of cryogenic catheter 240 includes one or more regions that can be selectively deflected so that the cryogenic catheter 240 is steerable. The cryogenic catheter 240 optionally include one or more electrodes 242 at the distal end portion of the cryogenic catheter 240. The electrodes 242 can be used to detect anatomical structures. The cryogenic catheter 240 also includes a micro-tube 244 by which coolant can be delivered to the distal end portion of the cryogenic catheter 240. In some examples, the distal tip portion of the cryogenic catheter 240 is uninsulated, while regions of the cryogenic catheter 240 proximal thereto are insulated.

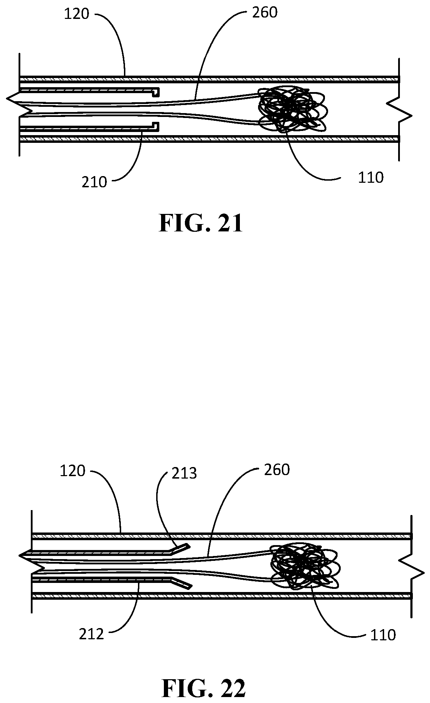

FIG. 21 depicted another example cryogenic catheter 260. The cryogenic catheter 260 comprises a looped micro-tube through which coolant can be passed. The cryogenic catheter 260 can be slidably disposed within a sheath 210. The distal end portion of the cryogenic catheter 260 forms a loop. The loop is the active portion of the cryogenic catheter 260 in that the loop is engageable with the thrombus 110 and can be cooled to dehydrate, freeze, and adhere to the thrombus 110. In some examples, the looped distal tip portion of the cryogenic catheter 260 is uninsulated, while regions of the cryogenic catheter 260 proximal thereto are insulated.

FIG. 22 depicts the cryogenic catheter 260 in another type of sheath 212. The sheath 212 includes a radially expandable distal end portion 213. The distal end portion 213 can be radially expanded in order to allow a large thrombus 110 to be pulled into the lumen of the sheath 212 using the cryogenic catheter 260. A clinician can control the sheath 212 to cause the radially expandable distal end portion 213 to expand and contract as desired. This type of sheath 212 can be used with any of the cryogenic catheters described herein.

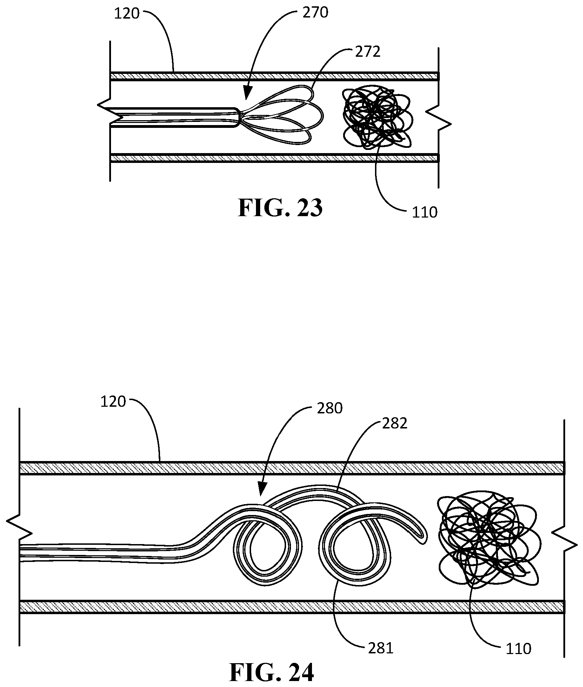

FIG. 23 depicts another example cryogenic catheter 270. The distal end portion of the cryogenic catheter 270 comprises multiple loops of micro-tube 272 through which coolant can be passed. The multiple loops of micro-tube 272 is the active portion of the cryogenic catheter 270 in that the loops are engageable with the thrombus 110 and can be cooled to dehydrate, freeze, and adhere to the thrombus 110. In some examples, the looped distal tip portion 272 of the cryogenic catheter 270 is uninsulated, while regions of the cryogenic catheter 270 proximal thereto are insulated. Optionally, the cryogenic catheter 270 can be delivered to adjacent the thrombus 110 in a sheath (e.g., sheath 210 or 212) and then expressed distally therefrom so that the multiple loops of micro-tube 272 can become engaged with the thrombus 110.

FIG. 24 depicts another example cryogenic catheter 280. The cryogenic catheter 280 includes a catheter shaft 281 that contains a micro-tube 282. The distal end portion of the catheter shaft 281 comprises multiple loops. The micro-tube 282 provides a flow path through which coolant can be passed. The distal end portion of the catheter shaft 281 is the active portion of the cryogenic catheter 280 in that its loops are engageable with the thrombus 110 and can be cooled to dehydrate, freeze, and adhere to the thrombus 110. In some examples, the distal end portion of the catheter shaft 281 of the cryogenic catheter 280 is uninsulated, while regions of the cryogenic catheter 280 proximal to the distal end portion of the catheter shaft 281 are insulated. Optionally, the cryogenic catheter 280 can be delivered to adjacent the thrombus 110 in a sheath (e.g., sheath 210 or 212) and then expressed distally therefrom so that the distal end portion of the catheter shaft 281 can become engaged with the thrombus 110. In some examples, the distal end portion of the catheter shaft 281 is essentially linear while in the sheath, and then (e.g., by virtue of shape-memory) forms the loops when expressed from the sheath.

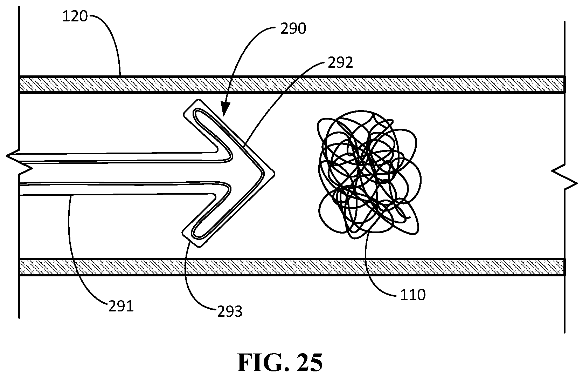

FIG. 25 depicts another example cryogenic catheter 290. The cryogenic catheter 290 includes a catheter shaft 291 that contains a micro-tube 292. The distal end portion of the catheter shaft 291 comprises an expandable basket 293. The micro-tube 292 provides a flow path through which coolant can be passed. The distal end portion of the catheter shaft 291 with its expandable basket 293 is the active portion of the cryogenic catheter 290 in that its loops are engageable with the thrombus 110 and can be cooled to dehydrate, freeze, and adhere to the thrombus 110. In some examples, the expandable basket 293 of the cryogenic catheter 290 is uninsulated, while regions of the cryogenic catheter 290 proximal to the expandable basket 293 are insulated. Optionally, the cryogenic catheter 290 can be delivered to adjacent the thrombus 110 in a sheath (e.g., sheath 210 or 212) and then expressed distally therefrom so that the distal end portion of the catheter shaft 291 can become engaged with the thrombus 110. In some examples, the radial configuration of the expandable basket 293 is controllable by a clinician. That is, a clinician can actuate the expandable basket 293 to radially expand or radially contract.

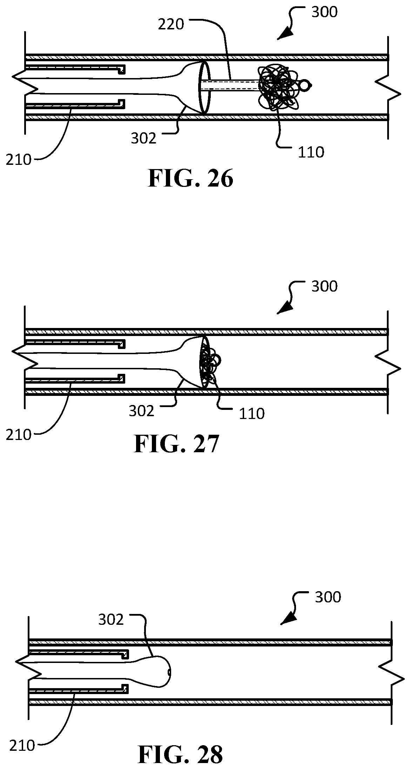

FIG. 26 depicts another example cryogenic catheter system 300. The catheter system 300 includes the guide sheath 210, the cryogenic catheter 220, and a thrombus capture and containment sheath 302. The distal end portion of the thrombus capture and containment sheath 302 is selectively diametrically expandable and retractable. In some examples, the distal end portion of the thrombus capture and containment sheath 302 is retractable using a lasso wire that encircles the distal end. In particular examples, the distal end portion of the thrombus capture and containment sheath 302 is retractable by in-folding.

The cryogenic catheter 220 is passed through the thrombus 110. Then the cryogenic catheter 220 is cryogenically activated to adhere the thrombus 110 to the cryogenic catheter 220. Next, the cryogenic catheter 220 is pulled proximally into the containment sheath. Next, the lasso wire (which extends to the proximal portion of the sheath), is retracted leading to infolding and closure of the containment sheath 302. The containment sheath is then removed en-bloc (as a whole) from the body while the guiding sheath 210 remain in place to allow repeating of the procedure for additional thrombus removal.

FIG. 27 shows the thrombus 110 when it has been pulled proximally to position the thrombus 110 within the expanded distal end portion of the thrombus capture and containment sheath 302.

FIG. 28 shows the thrombus capture and containment sheath 302 after the distal end portion has been diametrically retracted to capture and contain the thrombus 110 therein. Then, the guide sheath 210 and the thrombus capture and containment sheath 302 can be withdrawn from the vessel.

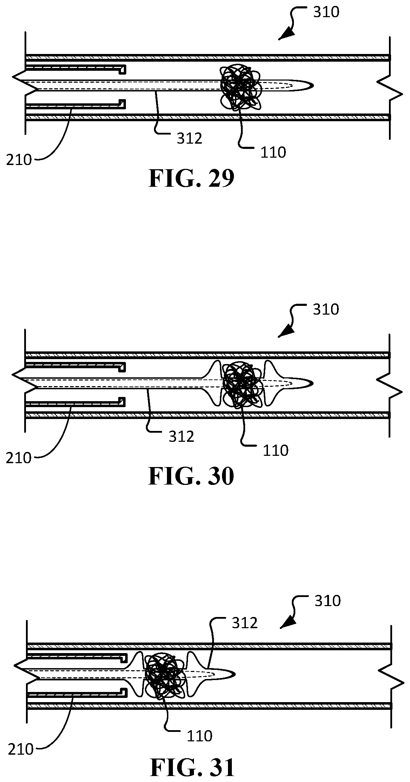

FIG. 29 depicts another example cryogenic catheter system 310. The cryogenic catheter system 310 includes the guide sheath 210 and a diametrically expandable cryogenic catheter 312. The diametrically expandable cryogenic catheter 312 includes an expandable distal portion and an expandable proximal portion. A cryogenic portion is located between the expandable distal portion and the expandable proximal portion.

To use the cryogenic catheter system 310, first the diametrically expandable cryogenic catheter 312 is passed through the thrombus 110, as depicted in FIG. 29. Then the cryogenic portion is activated (cooled) to cause adherence of the thrombus 110 to the cryogenic catheter 312.

As shown in FIG. 30, the expandable distal portion and the expandable proximal portion of the diametrically expandable cryogenic catheter 312 are then diametrically expanded. Next, as shown in FIG. 31 the diametrically expandable cryogenic catheter 312 is pulled proximally, and then the diametrically expandable cryogenic catheter 312 and the thrombus 110 can be removed from the vessel. The guide sheath 210 can remain in place for repeated procedures or withdrawn from the vessel along with the cryogenic catheter 312.

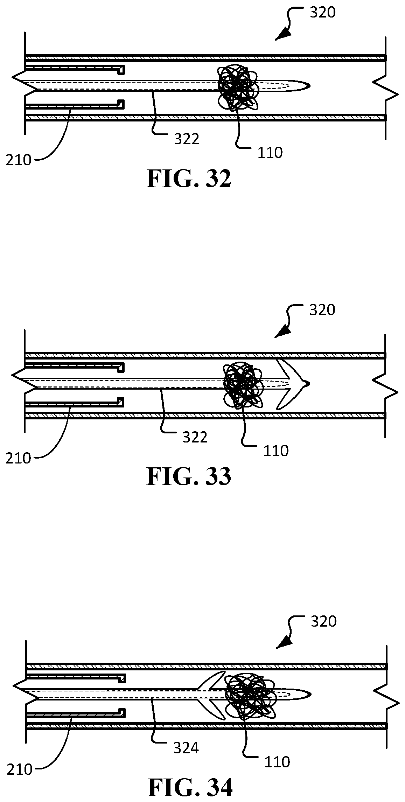

FIGs. 32 and 33 depicts another example cryogenic catheter system 320. The cryogenic catheter system 320 includes the guide sheath 210 and a basket catheter 322. The basket catheter 322 includes a cryogenic portion and an energy-activated expandable basket at the distal end of the basket catheter 322. In some examples, the basket catheter 322 can deliver a DC charge to cause fibrinogen to change its conformation and hence allow the basket at the distal end of the basket catheter 322 to act like a filter to capture the thrombus 110 in the basket. Then, the basket catheter 322 and the guide sheath 210 can be withdrawn from the vessel.

FIG. 34 depicts a modification of the cryogenic catheter system 320. In this example a basket catheter 324 is included that is analogous to the basket catheter 322 except that the energy-activated expandable basket is located proximally.

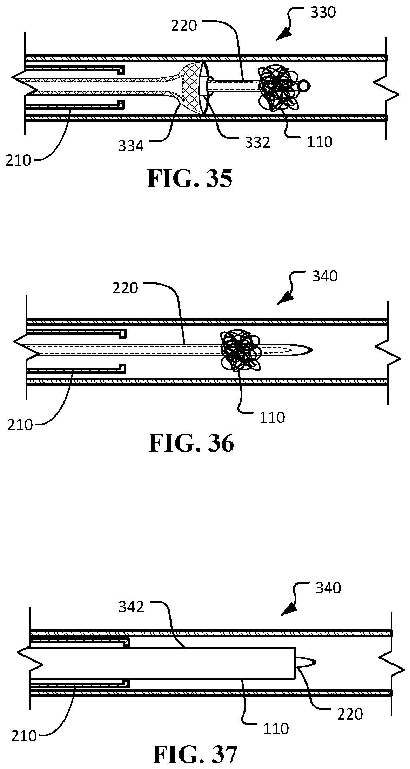

FIG. 35 depicts another example cryogenic catheter system 330. The cryogenic catheter system 330 includes the guide sheath 210, the cryogenic catheter 220, and an inner aspiration catheter 332, and an expandable cryo-enabled outer sheath 334. The cryogenic catheter 220 is slidably disposed in the inner aspiration catheter 332, and the inner aspiration catheter 332 is slidably disposed in the expandable cryo-enabled outer sheath 334.

To use the cryogenic catheter system 330, first the cryogenic catheter 220 is pushed just proximal to or through the thrombus 110 and then the cryogenics are activated to cause the thrombus 110 to adhere to the cryogenic catheter 220. The distal end portion of the expandable cryo-enabled outer sheath 334 is diametrically expanded. In addition, the cryogenics of the cryo-enabled outer sheath 334 are activated. Next, the cryogenic catheter 220 is pulled proximally and the thrombus 110 is captured by the combination of the cryogenic catheter 220 and the expandable cryo-enabled outer sheath 334. The inner aspiration catheter 332 can be in use to provide suction to aspirate particulate during portion of the process or the entire process to provide protection from distal embolization.

FIGs. 36 and 37 depicts another example cryogenic catheter system 340. The cryogenic catheter system 340 includes the guide sheath 210, the cryogenic catheter 220, and an outer sheath 342. The cryogenic catheter 220 is slidably disposed within the outer sheath 342. To use the cryogenic catheter system 340, first the cryogenic catheter 220 is pushed through the thrombus 110 and then the cryogenics are activated to cause the thrombus 110 to adhere to the cryogenic catheter 220. Next, the outer sheath 342 can be advanced (and/or the cryogenic catheter 220 with the thrombus 110 can be pulled proximally) until the thrombus 110 is inside of the outer sheath 342. Then, outer sheath 342 containing the cryogenic catheter 220 with the thrombus 110, and the guide sheath 210 can be pulled proximally to with them from the vessel.

In some examples, a distal end portion of the outer sheath 342 is expandable, or a portion of the distal end portion of the outer sheath 342 is expandable. In some such examples, a portion of the outer sheath 342 is diaphragm expandable. In some such examples, the distal end portion of the outer sheath 342 is expandable into a funnel shape.

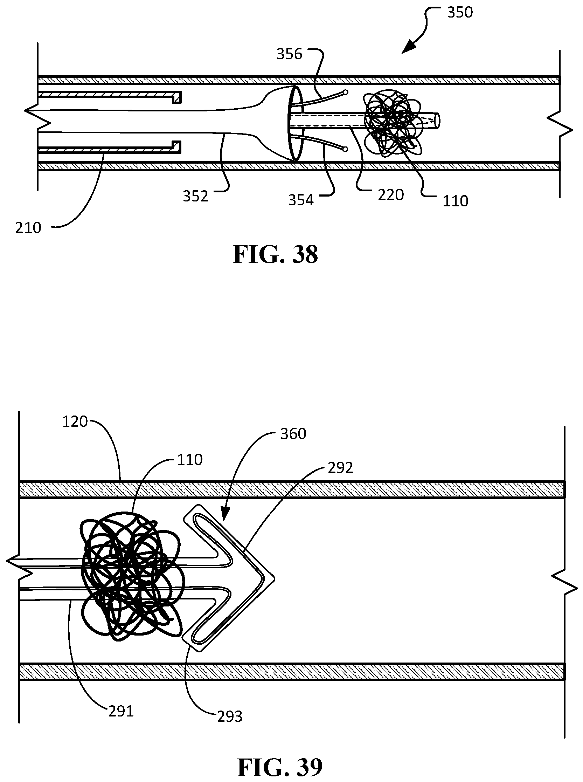

FIG. 38 depicts another example cryogenic catheter system 350. The cryogenic catheter system 350 includes the guide sheath 210, the cryogenic catheter 220, a diametrically expandable sheath 352, a first cryogenic catheter 354, and a second cryogenic catheter 356 that is optional. The cryogenic catheter 220, the first cryogenic catheter 354, and the second cryogenic catheter 356 are each slidably disposed within the diametrically expandable sheath 352. A distal end portion of the diametrically expandable sheath 352 is diametrically expandable into the shape of a funnel. In some examples, the diametrically expandable sheath 352 defines multiple lumens in which the cryogenic catheter 220, the first cryogenic catheter 354, and the second cryogenic catheter 356 are each individually slidably disposed within the diametrically expandable sheath 352. To use the cryogenic catheter system 350, first the cryogenic catheter 220 is pushed through the thrombus 110 and then the cryogenics are activated to cause the thrombus 110 to adhere to the cryogenic catheter 220. Next, the cryogenic catheter 220 with the adhered thrombus 110 is pulled proximally into the expanded distal end portion of the diametrically expandable sheath 352. The first cryogenic catheter 354 and the optional second cryogenic catheter 356 can be used to help manipulate the thrombus 110 into the expanded distal end portion of the diametrically expandable sheath 352.

FIG. 39 depicts another example cryogenic catheter system 360. The cryogenic catheter system 360 includes a catheter shaft 291 that contains a micro-tube 292. The distal end portion of the catheter shaft 291 comprises an expandable basket 293. The micro-tube 292 provides a flow path through which coolant can be passed. The distal end portion of the catheter shaft 291 with its expandable basket 293 is the active portion of the cryogenic catheter 290 in that its loops are engageable with the thrombus 110 and can be cooled to dehydrate, freeze, and adhere to the thrombus 110. In some examples, the expandable basket 293 of the cryogenic catheter 290 is uninsulated, while regions of the cryogenic catheter 290 proximal to the expandable basket 293 are insulated. Optionally, the cryogenic catheter 290 can be delivered to adjacent the thrombus 110 in a sheath (e.g., sheath 210 or 212) and then expressed distally therefrom so that the distal end portion of the catheter shaft 291 can become engaged with the thrombus 110. In some examples, the radial configuration of the expandable basket 293 is controllable by a clinician. That is, a clinician can actuate the expandable basket 293 to cause it to radially expand or radially contract.

The systems and methods described herein can be used for any and all types of intravascular clot or embolus removal treatment such as, but not limited to, pulmonary embolism or thrombosis, deep venous embolism or thrombosis, arterial embolism or thrombosis, coronary embolism or thrombosis, bypass graft embolism thrombosis, renal vein embolism or thrombosis, portal vein embolism or thrombosis, intracardiac embolism thrombosis, carotid and cerebral vessel embolism or thrombosis.

While this specification contains many specific implementation details, these should not be construed as limitations on the scope of any invention or of what may be claimed, but rather as descriptions of features that may be specific to particular embodiments of particular inventions. Certain features that are described in this specification in the context of separate embodiments can also be implemented in combination in a single embodiment. Conversely, various features that are described in the context of a single embodiment can also be implemented in multiple embodiments separately or in any suitable subcombination.

Similarly, while operations are depicted in the drawings in a particular order, this should not be understood as requiring that such operations be performed in the particular order shown or in sequential order, or that all illustrated operations be performed, to achieve desirable results. In certain circumstances, multitasking and parallel processing may be advantageous. Moreover, the separation of various system modules and components in the embodiments described herein should not be understood as requiring such separation in all embodiments, and it should be understood that the described program components and systems can generally be integrated together in a single product or packaged into multiple products.

Particular embodiments of the subject matter have been described. Other embodiments are within the scope of the following claims.

As one example, the processes depicted in the accompanying figures do not necessarily require the particular order shown, or sequential order, to achieve desirable results. In certain implementations, multitasking and parallel processing may be advantageous.

## Claims

1. A blood clot retrieval system comprising:
a cryogenic catheter (200) defining a coolant flow loop (224), the cryogenic catheter (200) configured for intravascular use; and
a wire (222) that is slidably disposable within a lumen of the cryogenic catheter (200) and distally extendable from a distal tip of the cryogenic catheter (200),
wherein the coolant flow loop (224) is arranged to cool at least a distal end portion of the wire (222) when a coolant is within the coolant flow loop (224) and the wire (222) is within the lumen.

2. The blood clot retrieval system of claim 1, wherein the distal end portion of the wire comprises a spiral.

3. The blood clot retrieval system of claim 1, wherein the distal end portion of the wire comprises a corkscrew configuration.

4. The blood clot retrieval system of claim 1, wherein the distal end portion of the wire comprises multiple separate loops.

5. The blood clot retrieval system of claim 1, wherein the distal end portion of the wire comprises a basket configuration.

6. A blood clot retrieval system comprising:
a catheter (250) configured for intravascular use;
a wire (252) that is slidably disposable within a lumen of the catheter (250); and
at least two Peltier devices (254) attached to a distal end portion of the wire (252) and spaced apart from each other, wherein activation of the Peltier devices (254) cools a first portion of the wire (252) located between the Peltier devices (254) and heats a second portion of the wire (252) located between the Peltier devices (254), wherein the first portion of the wire (252) is uninsulated and the second portion of the wire (252) is insulated.

7. The blood clot retrieval system of claim 6, wherein the distal end portion of the wire comprises a spiral.

8. The blood clot retrieval system of claim 6, wherein the distal end portion of the wire comprises a corkscrew configuration.

9. The blood clot retrieval system of claim 6, wherein the distal end portion of the wire comprises multiple separate loops.

## Patentansprüche

1. Blutgerinnselentfernungssystem, umfassend:
einen kryogenen Katheter (200), der eine Kühlmittelströmungsschleife (224) definiert, wobei der kryogene Katheter (200) zur intravaskulären Verwendung ausgelegt ist, und
einen Draht (222), der verschiebbar in einem Lumen des kryogenen Katheters (200) angeordnet und distal von einer distalen Spitze des kryogenen Katheters (200) ausfahrbar ist,
wobei die Kühlmittelströmungsschleife (224) dazu ausgelegt ist, mindestens einen distalen Endabschnitt des Drahts (222) zu kühlen, wenn sich ein Kühlmittel in der Kühlmittelströmungsschleife (224) befindet und sich der Draht (222) in dem Lumen befindet.

2. Blutgerinnselentfernungssystem nach Anspruch 1, wobei der distale Endabschnitt des Drahts eine Spirale umfasst.

3. Blutgerinnselentfernungssystem nach Anspruch 1, wobei der distale Endabschnitt des Drahts eine Korkenzieherkonfiguration umfasst.

4. Blutgerinnselentfernungssystem nach Anspruch 1, wobei der distale Endabschnitt des Drahts mehrere separate Schleifen umfasst.

5. Blutgerinnselentfernungssystem nach Anspruch 1, wobei der distale Endabschnitt des Drahts eine Korbkonfiguration umfasst.

6. Blutgerinnselentfernungssystem, umfassend:
einen zur intravaskulären Verwendung ausgelegten Katheter (250),
einen Draht (252), der verschiebbar in einem Lumen des Katheters (250) angeordnet werden kann, und
mindestens zwei Peltier-Vorrichtungen (254), die an einem distalen Endabschnitt des Drahts (252) angebracht und voneinander beabstandet sind, wobei durch die Aktivierung der Peltier-Vorrichtungen (254) ein sich zwischen den Peltier-Vorrichtungen (254) befindender erster Abschnitt des Drahts (252) gekühlt wird und ein sich zwischen den Peltier-Vorrichtungen (254) befindender zweiter Abschnitt des Drahts (252) erwärmt wird, wobei der erste Abschnitt des Drahts (252) nicht isoliert ist und der zweite Abschnitt des Drahts (252) isoliert ist.

7. Blutgerinnselentfernungssystem nach Anspruch 6, wobei der distale Endabschnitt des Drahts eine Spirale umfasst.

8. Blutgerinnselentfernungssystem nach Anspruch 6, wobei der distale Endabschnitt des Drahts eine Korkenzieherkonfiguration umfasst.

9. Blutgerinnselentfernungssystem nach Anspruch 6, wobei der distale Endabschnitt des Drahts mehrere separate Schleifen umfasst.

## Revendications

1. Système de récupération de caillots de sang comprenant :
un cathéter cryogénique (200) définissant une boucle d'écoulement de réfrigérant (224), le cathéter cryogénique (200) étant configuré pour une utilisation intravasculaire ; et
un fil (222) pouvant être disposé de manière coulissante dans une lumière du cathéter cryogénique (200) et extensible de manière distale à partir d'une extrémité distale du cathéter cryogénique (200),
la boucle d'écoulement de réfrigérant (224) étant agencée pour refroidir au moins une partie d'extrémité distale du fil (222) lorsqu'un réfrigérant se trouve à l'intérieur de la boucle d'écoulement de réfrigérant (224) et que le fil (222) se trouve à l'intérieur de la lumière.

2. Système de récupération de caillots de sang selon la revendication 1, la partie d'extrémité distale du fil comprenant une spirale.

3. Système de récupération de caillots de sang selon la revendication 1, la partie d'extrémité distale du fil comprenant une configuration de tire-bouchon.

4. Système de récupération de caillots de sang selon la revendication 1, la partie d'extrémité distale du fil comprenant de multiples boucles séparées.

5. Système de récupération de caillots de sang selon la revendication 1, la partie d'extrémité distale du fil comprenant une configuration de panier.

6. Système de récupération de caillots de sang comprenant :
un cathéter (250) configuré pour une utilisation intravasculaire ;
un fil (252) pouvant être disposé de manière coulissante dans une lumière du cathéter (250) ; et
au moins deux dispositifs Peltier (254) fixés à une partie d'extrémité distale du fil (252) et espacés l'un de l'autre, l'activation des dispositifs Peltier (254) refroidissant une première partie du fil (252) située entre les dispositifs Peltier (254) et chauffant une seconde partie du fil (252) située entre les dispositifs Peltier (254), la première partie du fil (252) étant isolée et la seconde partie du fil (252) étant isolée.

7. Système de récupération de caillots de sang selon la revendication 6, la partie d'extrémité distale du fil comprenant une spirale.

8. Système de récupération de caillots de sang selon la revendication 6, la partie d'extrémité distale du fil comprenant une configuration de tire-bouchon.

9. Système de récupération de caillots de sang selon la revendication 6, la partie d'extrémité distale du fil comprenant de multiples boucles séparées.
